# EUROPEAN PATENT APPLICATION

(11) **EP 3 375 776 A1**
(43) Date of publication of application: **19.09.2018**
(21) Application number: 16812780.1
(22) Date of filing: 08.11.2016
(51) Int. Cl.: C07D 233/58, C09K 5/06

(54) **BISIMIDAZOLIUM SALTS FOR ENERGY STORAGE**

(30) Priority: 10.11.2015 ES 201531615
(71) Applicant: Universitat de Lleida, 25003 Lérida (ES)
(72) Inventor: CANELA GARAYOA, Ramón, E- 25003 Lleida (ES); BALCELLS FLUVIÀ, Mercè, E- 25003 Lleida (ES); ERAS JOLI, Jordi, E- 25003 Lleida (ES); SALA MARTÍ, Nuria, E- 25003 Lleida (ES); YARA VARON, Edinson, E- 25003 Lleida (ES); ESCRIBÀ GELONCH, Marc, E- 25003 Lleida (ES); BARRENECHE GÜERISOLI, Camila, E- 25003 Lleida (ES); MARTORELL BOADA, Ingrid, E- 25003 Lleida (ES); CABEZA FABRA, Luisa Fernanda, E- 25003 Lleida (ES)
(74) Representative: ABG Patentes, S.L.
(86) International application number: PCT/ES2016/070790
(87) International publication number: WO 2017/081343

(57) **Abstract**

The invention relates to the use of compounds of formula (I) in thermal energy storage, to energy storage devices comprising said compounds of formula (I), as well as to compounds of formula (I) and the method for obtaining same.

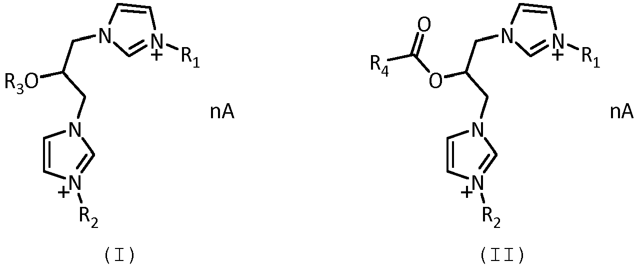

## Description

### Field of the Invention

The present invention relates to bisimidazolium salts, to the use thereof in thermal energy storage, to energy storage devices comprising said salts and to the method for obtaining same.

### Background of the Invention

Energy storage technologies are a key component for an efficient use of energy resources. The purpose of these technologies is to replace fossil fuel combustion with the use of stored heat which would be wasted otherwise, and also the use of renewable resources. Thermal energy storage can also be used for cooling in order to reduce or eliminate electricity consumption.

In this context, phase change materials are an appealing alternative due to their high energy conservation efficiency. These materials store and release large amounts of energy; specifically, heat is absorbed or released when the material changes state, particularly from solid to liquid and vice versa. For this reason, phase change materials are classified as latent heat storage units. The energy absorption-release cycle of phase change materials is as follows: the temperature of the phase change materials increases as they absorb heat; when they reach the temperature at which they change phase (their melting temperature), they absorb large amounts of heat at an almost constant temperature; the phase change material continues to absorb heat without a significant increase in temperature until all the material is transformed into liquid phase; finally, when the room temperature around said material (in liquid state) drops, the phase change material solidifies, releasing the stored latent heat.

There is a variety of phase change materials, including both organic and inorganic materials. Generally, inorganic materials have the drawback of being chemically unstable and highly corrosive, whereas organic materials tend to have problems relating to their low decomposition temperature, low thermal conductivity, flammability and low energy storage density.

To solve these drawbacks, the use of ionic liquids containing organic cations and inorganic anions has been proposed. However, many of these compounds have a low thermal energy storage density.

Ionic liquids are salts generally made up of an organic cation containing a heteroatom (N or P) associated with an organic or inorganic anion. The advantages of compounds of this type are their low melting point (compared to other salts), low vapor pressure, high thermal stability and high specific heat.

Imidazolium salt-based ionic liquids have been described in applications relating to energy storage. In this sense, MacFarlane et al. (Energy Environ. Sci., 2014, 7, 232-250) describe the use of 1,2-dialkylmethylimidazolium, a monoimidazolium salt, in energy applications. International patent application WO2013/182713A1 discloses the ionic liquids 1,7-bis(3-methylimidazolium-1-yl)heptane bromide and 1,8-bis(3-methylimidazolium-1-yl)octane bromide as components of thermal fluids but the function of which is to modify the viscosity of said thermal fluids. Zhang et al. (Energy Fuels, 2014, 28, 2802-2810) describe the use of monoimidazolium salt-based ionic liquids as phase change materials for energy storage.

Bisimidazolium salt-based ionic liquids have also been disclosed in the state of the art, but the use of said bisimidazolium salts is in applications unrelated to energy storage. In this sense, Straubinger et al. (J. Organomet. Chem., 2011, 696, 687-692) disclose several bisimidazolium salts, such as dibromide salts and di(hexafluorophosphate) salts of 1,1'-(2-hydroxy-1,3-propanediyl)bis(3-methyl-1H-imidazolium), 1,1'-(2-hydroxy-1,3-propanediyl)bis(3-isopropyl-1H-imidazolium) and 1,1'-(2-hydroxy-1,3-propanediyl)bis(3-benzyl-1H-imidazolium), for example. However, said document only refers to the use of said compounds in the formation of rhodium (I) complexes for use thereof in catalytic hydrosilylation reactions. Mandai et al. (Bull. Chem. Soc. Jpn., 2012, 85, 599-605) study the crystalline and thermal properties of bismethylimidazolium salts bound by alkylene chains having 4 to 8 linkages without steric hindrance, suggesting the use thereof as a stationary phase in gas chromatography, a solvent for high temperature organic reactions and high temperature lubricants. Yara-Varón et al. (RSC Adv., 2014, 4, 38418-38424) disclose chloride and hexafluorophosphate salts of 1,1'-(2-acroyloxy-1,3-propanediyl)bis(3-butyl-1H-imidazolium) and the preparation thereof from the intermediate 1,1'-(2-hydroxy-1,3-propanediyl)bis(3-butyl-1H-imidazolium) dichloride. However, said document only describes the use of the acrylate derivative as a monomer in the formation of polymeric biocatalyst materials.

There is a need to provide new phase change materials for energy storage which do not have the drawbacks of inorganic phase change materials, but which at the same time have good energy storage capacity, and which particularly can be obtained from renewable raw materials, thereby reducing greenhouse gas emission, pollutants, processing and the use of petroleum and petrochemical products.

### Summary of the Invention

The inventors have discovered that bisimidazolium salts derived from glycerol have surprisingly high enthalpies of fusion and solidification, therefore rendering them suitable as phase change materials for thermal energy storage. Particularly, bisimidazolium salts have higher enthalpies of fusion and solidification than the corresponding monoimidazolium salts. Furthermore, said salts can be obtained from glycerol, for example from the biodiesel industry, and carboxylic acids derived from the waste generated in slaughterhouses, both being renewable raw materials. Furthermore, said bisimidazolium salts do not have the drawbacks described above for inorganic phase change materials.

In a first aspect, the present invention relates to the use of a bisimidazolium salt of formula (I) in thermal energy storage 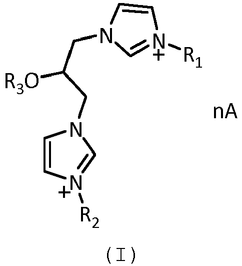 wherein
R₁ and R₂ are independently selected from the group consisting of linear or branched C₁-C₁₂ alkyl, wherein the C₁ alkyl is optionally substituted with one, two or three C₆-C₁₀ aryl groups; linear or branched C₂-C₁₂ alkenyl and C₆-C₁₀ aryl;
R₃ is selected from the group consisting of H and R₄CO-;
R₄ is selected from the group consisting of linear or branched C₁-C₂₀ alkyl, wherein the C₁ alkyl is optionally substituted with one, two or three C₆-C₁₀ aryl groups; linear or branched C₂-C₂₀ alkenyl and C₆-C₁₀ aryl;
A is a monovalent or divalent anion; and
n is 2 when A is a monovalent anion or n is 1 when A is a divalent anion.

In a second aspect, the present invention relates to a thermal energy storage device comprising a compound of formula (I) as defined in the first aspect.

In a third aspect, the present invention relates to a compound of formula (II) 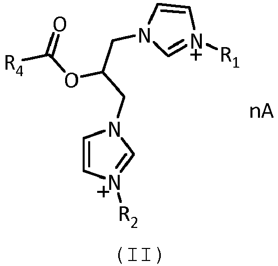 wherein
R₁ and R₂ are independently selected from the group consisting of linear or branched C₁-C₁₂ alkyl, wherein the C₁ alkyl is optionally substituted with one, two or three C₆-C₁₀ aryl groups, linear or branched C₂-C₁₂ alkenyl and C₆-C₁₀ aryl;
R₄ is selected from the group consisting of linear or branched C₁-C₂₀ alkyl, wherein the C₁ alkyl is optionally substituted with one, two or three C₆-C₁₀ aryl groups, linear or branched C₃-C₂₀ alkenyl and C₆-C₁₀ aryl;
A is a monovalent or divalent anion; and
n is 2 when A is a monovalent anion or n is 1 when A is a divalent anion,
where the compound in which R₁ and R₂ are ethyl, R₄ is -C(CH₃)=CH₂ and A is (CF₃SO₂)₂N⁻ is excluded from formula (II) .

In a fourth aspect, the present invention relates to a method for obtaining a compound of formula (II) as defined in the third aspect which comprises:
a) reacting glycerol with a carboxylic acid of formula (III) in the presence of a chlorinating agent

   R₄-COOH (III)

   to yield a compound of formula (IV) 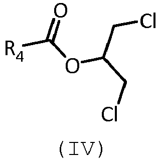 wherein R₄ is as defined in the third aspect,
b) reacting the compound of formula (IV) with a compound of formula (V) and a compound of formula (VI), 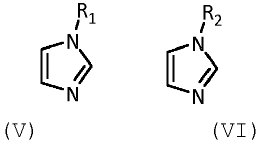
   wherein R₁ and R₂ are as defined in the third aspect, and when R₁=R₂, then compounds (V) and (VI) are the same compound;
   to yield a compound of formula (II), 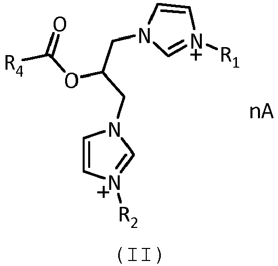
   wherein R₁, R₂ and R₄ are as defined in the third aspect; n is 2 and A is Cl⁻;
c) optionally treating the compound of formula (II) with a salt of A to yield a compound of formula (II) wherein n is as defined in the third aspect and A is a monovalent or divalent anion as defined in the third aspect other than Cl⁻.

### Detailed Description of the Invention

In a first aspect, the invention relates to the use of a compound of formula (I) in thermal energy storage 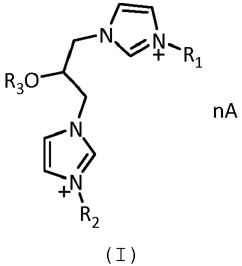 wherein
R₁ and R₂ are independently selected from the group consisting of linear or branched C₁-C₁₂ alkyl, wherein the C₁ alkyl is optionally substituted with one, two or three C₆-C₁₀ aryl groups; linear or branched C₂-C₁₂ alkenyl and C₆-C₁₀ aryl;
R₃ is selected from the group consisting of H and R₄CO-;
R₄ is selected from the group consisting of linear or branched C₁-C₂₀ alkyl, wherein the C₁ alkyl is optionally substituted with one, two or three C₆-C₁₀ aryl groups, linear or branched C₂-C₂₀ alkenyl and C₆-C₁₀ aryl;
A is a monovalent or divalent anion; and n is 2 when A is a monovalent anion or n is 1 when A is a divalent anion.

The compounds of formula (I) and also the compounds of formula (II) described herein absorb or release heat when they change from solid to liquid state and vice versa. The temperature of said compounds increases as they absorb heat; when they reach the temperature at which they change phase (their melting temperature), they absorb large amounts of heat at an almost constant temperature; they continue to absorb heat without a significant increase in temperature until all the compound is transformed into liquid phase; finally, when the room temperature around said material (in liquid state) drops, said compounds solidify, releasing stored latent heat. Therefore, high enthalpies of fusion and solidification allow obtaining materials with a large energy storage capacity. Particularly, the compounds of formula (I) and also the compounds of formula (II) described below have enthalpies of fusion greater than 106 kJ/kg, preferably between 110 and 1000 kJ/kg, more preferably between 170 and 1000 kJ/kg. Particularly, the compounds of formula (I) and also the compounds of formula (II) described below have enthalpies of solidification of at least 110 kJ/kg, preferably between 150 and 370 kJ/kg. These features of the compounds of formula (I) and also the compounds of formula (II) described below render them suitable for use in thermal energy storage, particularly in storing thermal energy in the form of latent heat.

In the context of the present invention, "alkyl" refers to a linear or branched hydrocarbon chain radical consisting of carbon and hydrogen atoms, containing no unsaturations, having the number of indicated carbon atoms, for example from 1 to 20 carbon atoms, from 10 to 20 carbon atoms, from 1 to 12 carbon atoms, from 1 to 6 carbon atoms, from 3 to 6 carbon atoms, and being bound to the rest of the molecule by means of a single bond. Examples of alkyl are methyl, ethyl, n-propyl, isopropyl, n-butyl, *sec*-butyl, iso-butyl, *tert*-butyl, n-pentyl, neopentyl, n-hexyl, n-heptyl, n-octyl, n-nonyl, n-decanyl, n-undecanyl, n-dodecanyl, n-tridecanyl, n-tetradecanyl, n-pentadecanyl, etc.

"Alkenyl" herein refers to a linear or branched hydrocarbon chain radical consisting of carbon and hydrogen atoms, containing at least one unsaturation, having the number of indicated carbon atoms, for example from 2 to 20 carbon atoms, from 3 to 20 carbon atoms, from 2 to 6 carbon atoms, from 3 to 6 carbon atoms, and being bound to the rest of the molecule by means of a single bond. Examples of alkenyl are vinyl, n-propenyl, i-propenyl, n-butenyl, n-pentenyl, etc.

"Aryl" herein refers to an aromatic hydrocarbon radical consisting of carbon and hydrogen atoms, having the number of indicated carbon atoms, such as from 6 to 10 carbon atoms, for example, and being bound to the rest of the molecule by means of a single bond. Examples of aryl are phenyl and naphthyl.

Examples of C₁ alkyl substituted with one, two or three C₆-C₁₀ aryl groups are phenylmethyl, diphenylmethyl and trinaphthylmethyl, among others. Said C₆-C₁₀ aryl substituents can be the same or different.

"Monovalent anion" herein refers to an inorganic or organic anion with a single negative charge which can form an ionic bond with a cation of an imidazolium derivative. Examples of monovalent anions are PF₆⁻, Cl⁻, Br⁻, I⁻, F⁻, BF₄⁻, NO₃⁻, NO₂⁻, SCN⁻, BrO₃⁻, IO₃⁻, HCO₃⁻, HCOO⁻, CH₃COO⁻, HSO₄⁻, HSO₃⁻ and H₂PO₃⁻.

"Divalent anion" herein refers to an inorganic or organic anion with two negative charges which can form an ionic bond with two cations of an imidazolium derivative. Examples of divalent anions are oxalate (COO)₂²⁻, malonate ⁻OOCCH₂COO⁻, tartrate ⁻OOC-CHOH-CHOH-COO⁻, CO₃²⁻, SO₄²⁻, SO₃²⁻ and HPO₃²⁻.

In a preferred embodiment of the use of the compounds of formula (I), R₁ and R₂ are the same. In an alternative embodiment, R₁ and R₂ are different.

In another preferred embodiment of the use of the compounds of formula (I), R₁ and R₂ are independently a linear or branched C₁-C₁₂ alkyl, preferably linear or branched C₁-C₆ alkyl, preferably R₁ and R₂ are independently selected from the group consisting of butyl, isopropyl, propyl, ethyl and methyl, more preferably they are both the same, even more preferably they are both butyl.

In another preferred embodiment of the use of the compounds of formula (I), R₃ is H. In another embodiment, R₃ is R₄CO.

In another embodiment of the use of the compounds of formula (I), R₃ is R₄CO and R₄ is selected from the group consisting of linear C₁₀-C₂₀ alkyl, branched C₃-C₆ alkyl, methyl (i.e., C₁ alkyl) substituted with one, two or three C₆-C₁₀ aryl groups, linear C₂-C₆ alkenyl, naphthyl and phenyl, preferably from the group consisting of tert-butyl, isobutyl, isopropyl, 1,1-dimethylpropyl, 1,1-dimethylbutyl, pentadecanyl, vinyl, ethenyl, 1-propenyl, diphenylmethyl, phenylmethyl, naphthyl and phenyl, more preferably from the group consisting of tert-butyl, pentadecanyl, vinyl and naphthyl.

In another embodiment of the use of the compounds of formula (I), R₃ is R₄CO and R₄ is selected from the group consisting of linear C₁₀-C₂₀ alkyl, branched C₃-C₆ alkyl, linear C₂-C₆ alkenyl, naphthyl and phenyl, preferably from the group consisting of *tert*-butyl, isobutyl, isopropyl, 1,1-dimethylpropyl, 1,1-dimethylbutyl, pentadecanyl, vinyl, ethenyl, 1-propenyl, naphthyl and phenyl, more preferably from the group consisting of *tert*-butyl, pentadecanyl, vinyl and naphthyl.

In a preferred embodiment of the use of the compounds of formula (I), n is 2 and A is a monovalent anion. Preferably, A is selected from the group consisting of PF₆⁻, Cl⁻, Br⁻ and BF₄⁻, more preferably from the group consisting of PF₆⁻ and Cl⁻, even more preferably A is Cl⁻. The selection of Cl⁻ as monovalent anion A yields compounds with higher enthalpies of fusion and solidification.

In an alternative embodiment of the use of the compounds of formula (I), n is 1 and A represents a divalent anion.

In a particular embodiment of the use of the compounds of formula (I), R₁ and R₂ are independently a linear or branched C₁-C₆ alkyl; R₃ is selected from the group consisting of H and R₄CO-; R₄ is selected from the group consisting of linear C₁₀-C₂₀ alkyl, branched C₃-C₆ alkyl, linear C₂-C₆ alkenyl, naphthyl and phenyl; n is 2 and A represents a monovalent anion selected from the group consisting of PF₆⁻ and Cl⁻, preferably Cl⁻.

In another particular embodiment of the use of the compounds of formula (I), R₁ and R₂ are independently selected from the group consisting of butyl, isopropyl, propyl, ethyl and methyl; R₃ is selected from the group consisting of H and R₄CO-; R₄ is selected from the group consisting of linear C₁₀-C₂₀ alkyl, branched C₃-C₆ alkyl, linear C₂-C₆ alkenyl, naphthyl and phenyl; n is 2 and A represents a monovalent anion selected from the group consisting of PF₆⁻ and Cl⁻, preferably Cl⁻.

In another particular embodiment of the use of the compounds of formula (I), R₁ and R₂ are butyl; R₃ is selected from the group consisting of H and R₄CO-; R₄ is selected from the group consisting of linear C₁₀-C₂₀ alkyl, branched C₃-C₆ alkyl, linear C₂-C₆ alkenyl, naphthyl and phenyl; n is 2 and A represents a monovalent anion selected from the group consisting of PF₆⁻ and Cl⁻, preferably Cl⁻.

In another particular embodiment of the use of the compounds of formula (I), R₁ and R₂ are butyl; R₃ is selected from the group consisting of H and R₄CO-; R₄ is selected from the group consisting of *tert*-butyl, pentadecanyl, vinyl and naphthyl; n is 2 and A represents a monovalent anion selected from the group consisting of PF₆⁻ and Cl⁻, preferably Cl⁻.

In another particular embodiment of the use of the compounds of formula (I), R₁ and R₂ are butyl; R₃ is selected from the group consisting of H and R₄CO-; R₄ is selected from the group consisting of *tert*-butyl, pentadecanyl, vinyl and naphthyl; n is 2 and A is Cl⁻.

The present invention also relates to any combination of the particular and preferred embodiments described above.

In another embodiment of the use of the compounds of formula (I), the compound of formula (I) is selected from the group consisting of:
- 1,1'-(2-(2,2-dimethylpropanoyloxy)-1,3-propanediyl)bis(3-butyl-1H-imidazolium)dichloride,
- 1,1'-(2-(2,2-dimethylpropanoyloxy)-1,3-propanediyl)bis(3-butyl-1H-imidazolium)di(hexafluoride),
- 1,1'-(2-palmitoyloxy-1,3-propanediyl)bis(3-butyl-1H-imidazolium)dichloride,
- 1,1'-(2-palmitoyloxy-1,3-propanediyl)bis(3-butyl-1H-imidazolium)di(hexafluoride),
- 1,1'-(2-hydroxy-1,3-propanediyl)bis(3-butyl-1H-imidazolium) dichloride,
- 1,1'-(2-hydroxy-1,3-propanediyl)bis(3-butyl-1H-imidazolium) di(hexafluoride),
- 1,1'-(2-(1-naphthoyloxy)-1,3-propanediyl)bis(3-butyl-1H-imidazolium)dichloride,
- 1,1'-(2-(1-naphthoyloxy)-1,3-(propanediyl)bis(3-butyl-1H-imidazolium)di(hexafluoride),
- 1,1'-(2-acroyloxy-1,3-propanediyl)bis(3-butyl-1H-imidazolium) dichloride, and
- 1,1'-(2-acroyloxy-1,3-propanediyl)bis(3-butyl-1H-imidazolium) di(hexafluoride).

Another particular embodiment of the invention relates to the use of the compounds of formula (II) as defined herein, representing a subgroup of the compounds of formula (I).

Particularly, the use of the compounds of formula (I) and of formula (II) described in the present invention in thermal energy storage can be applied for thermoregulation of thermal comfort, for active indoor heating and cooling applications, for domestic hot water, solar cooling, etc.

In another aspect, the present invention relates to a thermal energy storage device comprising a compound of formula (I) as defined above, preferably a thermal energy storage device, more preferably a thermal energy device selected from the group consisting of solar panels, boards for walls and ceilings (in construction, which allow stabilizing indoor temperature). Said device consists of, for example, a double pipe heat exchanger, shell and tube heat exchanger, plate heat exchanger, etc. They can also consist of an isolated, jacketed vessel/tank with an inner exchanger, etc.

Another aspect of the invention relates to a compound of formula (II) 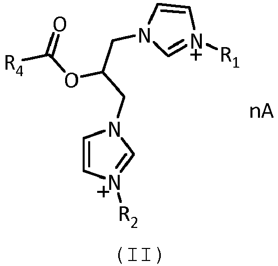 wherein
R₁ and R₂ are independently selected from the group consisting of linear or branched C₁-C₁₂ alkyl, wherein the C₁ alkyl is optionally substituted with one, two or three C₆-C₁₀ aryl groups; linear or branched C₂-C₁₂ alkenyl and C₆-C₁₀ aryl;
R₄ is selected from the group consisting of linear or branched C₁-C₂₀ alkyl, wherein the C₁ alkyl is optionally substituted with one, two or three C₆-C₁₀ aryl groups, linear or branched C₃-C₂₀ alkenyl and C₆-C₁₀ aryl;
A is a monovalent or divalent anion; and
n is 2 when A is a monovalent anion or n is 1 when A is a divalent anion;
where the compound in which R₁ and R₂ are ethyl, R₄ is -C(CH₃)=CH₂ and A is (CF₃SO₂)₂N⁻ is excluded from formula (II) .

In a preferred embodiment of the compounds of formula (II), R₁ and R₂ are the same. In an alternative embodiment, R₁ and R₂ are different.

In another preferred embodiment of the compounds of formula (II), R₁ and R₂ are independently a linear or branched C₁-C₂ alkyl, preferably R₁ and R₂ are independently a linear or branched C₁-C₆ alkyl, preferably R₁ and R₂ are independently selected from the group consisting of butyl, isopropyl, propyl, ethyl and methyl, more preferably they are both the same, even more preferably they are both butyl.

In another embodiment of the compounds of formula (II), R₄ is selected from the group consisting of linear or branched C₁-C₂₀ alkyl, wherein the C₁ alkyl is optionally substituted with one, two or three C₆-C₁₀ aryl groups; linear or branched C₃-C₂₀ alkenyl with the exception of the -C(CH₃)=CH₂ group; and C₆-C₁₀ aryl.

In another embodiment of the compounds of formula (II), R₄ is selected from the group consisting of linear or branched C₁-C₂₀ alkyl, wherein the C₁ alkyl is optionally substituted with one, two or three C₆-C₁₀ aryl groups; linear C₃-C₂₀ alkenyl, and C₆-C₁₀ aryl.

In another embodiment of the compounds of formula (II), R₄ is selected from the group consisting of linear or branched C₁-C₂₀ alkyl, wherein the C₁ alkyl is optionally substituted with one, two or three C₆-C₁₀ aryl groups; and C₆-C₁₀ aryl.

In another embodiment of the compounds of formula (II), R₄ is selected from the group consisting of linear C₁₀-C₂₀ alkyl, branched C₃-C₆ alkyl, methyl (i.e., C₁ alkyl) substituted with one, two or three C₆-C₁₀ aryl groups, linear C₃-C₆ alkenyl, naphthyl and phenyl; preferably from the group consisting of tert-butyl, isobutyl, isopropyl, 1,1-dimethylpropyl, 1,1-dimethylbutyl, 1-propenyl, diphenylmethyl, phenylmethyl, pentadecanyl, naphthyl and phenyl, more preferably from the group consisting of *tert*-butyl, pentadecanyl and naphthyl.

In another embodiment of the compounds of formula (II), R₄ is selected from the group consisting of linear C₁₀-C₂₀ alkyl, branched C₃-C₆ alkyl, naphthyl and phenyl, preferably from the group consisting of *tert*-butyl, isobutyl, isopropyl, 1,1-dimethylpropyl, 1,1-dimethylbutyl, pentadecanyl, naphthyl and phenyl, more preferably from the group consisting of *tert*-butyl, pentadecanyl, vinyl and naphthyl.

In one embodiment of the compounds of formula (II), n is 2 and A is a monovalent anion. Preferably, A is selected from the group consisting of PF₆⁻, Cl⁻, Br⁻ and BF₄⁻, more preferably from the group consisting of PF₆⁻ and Cl⁻, even more preferably A is Cl⁻. The selection of Cl⁻ as monovalent anion A yields compounds with higher enthalpies of fusion and solidification.

In an alternative embodiment of the compounds of formula (II), n is 1 and A represents a divalent anion.

In a particular embodiment of the compounds of formula (II), R₁ and R₂ are independently a linear or branched C₁-C₆ alkyl; R₄ is selected from the group consisting of linear C₁₀-C₂₀ alkyl, branched C₃-C₆ alkyl, naphthyl and phenyl; n is 2 and A represents a monovalent anion selected from the group consisting of PF₆⁻ and Cl⁻, preferably Cl⁻.

In another particular embodiment of the compounds of formula (II), R₁ and R₂ are independently selected from the group consisting of butyl, isopropyl, propyl, ethyl and methyl; R₄ is selected from the group consisting of linear C₁₀-C₂₀ alkyl, branched C₃-C₆ alkyl, naphthyl and phenyl; n is 2 and A represents a monovalent anion selected from the group consisting of PF₆⁻ and Cl⁻, preferably Cl⁻.

In another particular embodiment of the compounds of formula (II), R₁ and R₂ are butyl; R₄ is selected from the group consisting of linear C₁₀-C₂₀ alkyl, branched C₃-C₆ alkyl, naphthyl and phenyl; n is 2 and A represents a monovalent anion selected from the group consisting of PF₆⁻ and Cl⁻, preferably Cl⁻.

In another particular embodiment of the compounds of formula (II), R₁ and R₂ are butyl; R₄ is selected from the group consisting of *tert*-butyl, pentadecanyl and naphthyl; n is 2 and A represents a monovalent anion selected from the group consisting of PF₆⁻ and Cl⁻, preferably Cl⁻.

In another particular embodiment of the compounds of formula (II), R₁ and R₂ are butyl; R₄ is selected from the group consisting of *tert*-butyl, pentadecanyl and naphthyl; n is 2 and A is Cl⁻.

The present invention also relates to any combination of the particular and preferred embodiments described above.

In another embodiment of the compounds of formula (II), said compound is selected from the group consisting of:
- 1,1'-(2-(2,2-dimethylpropanoyloxy)-1,3-propanediyl)bis(3-butyl-1H-imidazolium)dichloride,
- 1,1'-(2-(2,2-dimethylpropanoyloxy)-1,3-propanediyl)bis(3-butyl-1H-imidazolium)di(hexafluoride),
- 1,1'-(2-palmitoyloxy-1,3-propanediyl)bis(3-butyl-1H-imidazolium)dichloride,
- 1,1'-(2-palmitoyloxy-1,3-propanediyl)bis(3-butyl-1H-imidazolium)di(hexafluoride),
- 1,1'-(2-(1-naphthoyloxy)-1,3-propanediyl)bis(3-butyl-1H-imidazolium)dichloride, and
- 1,1'-(2-(1-naphthoyloxy)-1,3-(propanediyl)bis(3-butyl-1H-imidazolium)di(hexafluoride).

The compounds of formula (II) are a subgroup of the compounds of formula (I) and are therefore also suitable for the uses described above with respect to the compounds of formula (I), i.e., in thermal energy storage.

Furthermore, in a particular embodiment the present invention also relates to a thermal energy storage device as defined above comprising a compound of formula (II), preferably a thermal energy storage device, more preferably a thermal energy device selected from the group consisting of solar panels, boards for walls and ceilings (in construction, which allow stabilizing indoor temperature). Said device consists of, for example, a double pipe heat exchanger, shell and tube heat exchanger, plate heat exchanger, etc. They can also consist of an isolated, jacketed vessel/tank with an inner exchanger, etc.

The compounds of formula (I) and the compounds of formula (II) can be obtained from renewable raw materials, such as glycerol, for example, from the biodiesel industry, for example, and carboxylic acids from slaughterhouses, for example.

Therefore, in another aspect the present invention relates to a method for obtaining a compound of formula (II) as defined above which comprises:
a) reacting glycerol with a carboxylic acid of formula (III) in the presence of a chlorinating agent

   R₄-COOH (III)

   to yield a compound of formula (IV) 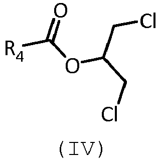 wherein R₄ is as defined above for the compounds of formula (II),
b) reacting the compound of formula (IV) with a compound of formula (V) and a compound of formula (VI), 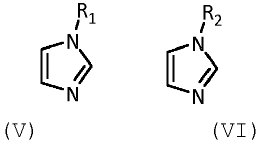
   wherein R₁ and R₂ are as defined above for the compounds of formula (II), and when R₁=R₂, then compounds (V) and (VI) are the same compound;
   to yield a compound of formula (II), 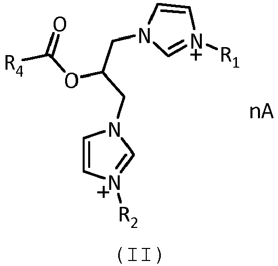 wherein R₁, R₂ and R₄ are as defined above for the compounds of formula (II); n is 2 and A is Cl⁻;
c)optionally treating the compound of formula (II) with a salt of A to yield a compound of formula (II) wherein n is as defined above for the compounds of formula (II) and A is a monovalent or divalent anion as defined above for the compounds of formula (II) other than Cl⁻.

Step a) of the method is an esterification-chlorination reaction. A chlorinating agent must be used to perform said step. Said chlorinating agent can be chlorotrimethylsilane, an ion-exchange resin (Amberlyst IR120H, Purolite PD206, Dowex DR-G8, Purolite C150) and a salt (for example, KCl or AlCl₃), a salt (for example, KCl and AlCl₃) in the presence of cyclodextrins or ionic liquids, mixtures of phosphoric acid and CaCl₂, AlCl₃ (particularly in the form of hexahydrate, i.e., AlCl₃·6H₂O) optionally in the presence of phosphoric acid, HCl gas, PCl₅, PCl₃, SOCl₂ [Eras J. et al., Journal of Organic Chemistry, 2002, 67, 8631-8634; Escriba M. et al., Tetrahedron, 2009, 65, 10370-10376; Escriba M. et al., Waste and Biomass Valorization, 2011, 2, 285-290; Oromi-Farrus M. et al., Analytical Sciences, 2008, 24, 1341-1345; Villorbina G. et al., Tetrahedron Letters, 2009, 50, 2828-2830; Escriba M. et al., RSC Advances, 2013, 3, 8805-8810]. Preferably, the chlorinating agent is selected from the group consisting of chlorotrimethylsilane, CaCl₂ in the presence of H₃PO₄ and AlCl₃ (particularly in the form of hexahydrate, i.e., AlCl₃·6H₂O) optionally in the presence of H₃PO₄. More preferably, the chlorinating agent is chlorotrimethylsilane.

Preferably, step a) is performed by means of heating (for example by means of conventional or microwave heating) between 60°C and 250°C, preferably between 75°C and 95°C, more preferably at 80°C, preferably in the absence of solvent (particularly when the chlorinating agent can act at the same time as reagent and solvent, like in the case of chlorotrimethylsilane). Solvents suitable for this step are aliphatic and aromatic hydrocarbons, such as hexane, heptane, cyclohexane, benzene, toluene, xylene, for example; eutectic solvents, such as mixtures of glycerol and choline chloride, glycerol and hydroxycholine, urea and choline chloride, thiourea and choline chloride, for example; ethers, such as diethyl ether, dioxane, tetrahydrofuran and methyltetrahydrofuran, for example; and halogenated solvents such as dichloromethane, chloroform, for example.

The time suitable for completing the reaction of step a) can be determined by the person skilled in the art by means of conventional methods, such as thin-layer chromatography (TLC), nuclear magnetic resonance (NMR) or high-performance liquid chromatography (HPLC), for example. Said time will depend on the heating system used. Particularly, the conventional heating is maintained for at least 30 h, preferably at least 40 h, more preferably at least 48 h, even more preferably between 40 h and 50 h. The microwave heating is preferably maintained for 5 min, more preferably 10 min, even more preferably between 15 min and 20 min.

The compound of formula (IV) obtained in step a) can be purified using conventional techniques such as extraction, chromatography, crystallization or distillation, for example.

Step b) of the method is preferably performed by means of heating between 80°C and 250°C, preferably between 100°C and 120°C, more preferably at 110°C, in the absence of solvent. The time suitable for completing the reaction can be determined by the person skilled in the art by means of conventional methods such as thin-layer chromatography (TLC), nuclear magnetic resonance (NMR) or high-performance liquid chromatography (HPLC), for example. In a particular embodiment, the heating is maintained for at least 30 h, preferably at least 40 h, more preferably at least 48 h, even more preferably between 40 h and 50 h. Preferably, the reaction is performed in inert atmosphere, such as argon or nitrogen atmosphere for example. The obtained compound of formula (II) can be purified using conventional techniques such as precipitation, extraction, chromatography, crystallization or distillation, for example.

In the particular case in which R₁ and R₂ in the compounds of formula (II) are the same, step b) of the method for obtaining said compounds which comprises reacting the compound of formula (IV) with a compound of formula (V) and a compound of formula (VI) which are the same compound, i.e., the compound of formula (V) and the compound of formula (VI) are identical since R₁ and R₂ are the same. Therefore, the way to react the compound of formula (IV) with a compound of formula (V) or the way to react a compound of formula (IV) with a compound of formula (VI) can also be considered.

Step c) of the method is optional and is performed when the compound of formula (II) has an anion A other than Cl⁻. Step c) is an anion exchange reaction. Said step comprises treating the compound of formula (II) in which A is Cl⁻ with a salt of A, preferably a metallic salt of A, more preferably a sodium or potassium salt of A, to yield a compound of formula (II), wherein A is a monovalent or divalent anion as defined above for the compounds of formula (II) other than Cl⁻. Preferably, said step is performed in an aqueous solution, more preferably the solvent is water. Said step is preferably performed at room temperature, i.e., between 20°C and 25°C. The time suitable for completing the reaction can be determined by the person skilled in the art by means of conventional methods such as thin-layer chromatography (TLC), nuclear magnetic resonance (NMR) or high-performance liquid chromatography (HPLC), for example. In a particular embodiment, the reaction is maintained between 5 and 30 min, more preferably between 5 min and 20 min, more preferably 15 min. The obtained compound of formula (II) can be isolated using conventional techniques such as extraction, for example.

The compounds of formula (I) wherein R₁ and R₂ are independently a linear or branched C₁-C₁₂ alkyl; R₃ is R₄CO-; R₄ is selected from the group consisting of linear or branched C₁-C₂₀ alkyl, linear or branched C₂-C₂₀ alkenyl and C₆-C₁₀ aryl; n is 1 or 2, as defined for the compounds of formula (I), and A represents a monovalent or divalent anion, as defined for the compounds of formula (I), can also be obtained by means of the method described above.

In the particular case in which R₃ is H in the compounds of formula (I), the method described in Straubinger et al. (J. Organomet. Chem., 2011, 696, 687-6929, incorporated herein by reference with respect to the synthesis methods described) can be used; said method comprises reacting 1,3-dibromopropan-2-ol with a compound of formula (V) or formula (VI) as described above depending on if R₁ and R₂ are the same or different, in tetrahydrofuran, at 100°C and in a pressure tube, to provide a compound of formula (I), wherein n is 2 and A represents Br⁻. The compounds of formula (I) wherein n is 1 or 2, as defined above for the compounds of formula (I), and A represents a monovalent or divalent anion other than Br⁻, as defined above for the compounds of formula (I), are obtained by anion exchange reaction, such as by reacting the bromide salt obtained with a salt of A as described in step c) of the method for obtaining the compounds of formula (II), preferably using water as solvent.

The following non-limiting examples seek to illustrate the present invention and must not be interpreted as limiting the scope of the present invention.

### Examples

### Materials and methods

### Materials and reagents

Acrylic acid, 1-naphthylcarboxylic acid, palmitic acid and pivalic acid were obtained from Sigma-Aldrich (Sigma-Aldrich Química, S.A., Madrid, Spain). Hexane, methanol, ethanol, acetone, acetonitrile, diethyl ether, isopropanol and dichloromethane were obtained from T.J. Baker (Quimega, Lleida, Spain). 1,3-dichloro-2-propanol, N,N-dicyclohexylcarbodiimide (DCC) and potassium hexafluorophosphate (KPF₆) were obtained from Across Organics (Barcelona, Spain). Potassium hydroxide was acquired from Panreac (Barcelona, Spain).

Crude glycerol was obtained from a biodiesel industrial supplier using an alkali-catalyzed alcoholysis method (Raluy, S.L., Spain). The crude glycerol was neutralized using sulfuric acid and most of the residual methanol was eliminated by vacuum distillation. Finally, the remaining material was centrifuged at 2600 g and decanted to eliminate solids in suspension. The final product was analyzed by ¹H-NMR in deuterated dimethylsulfoxide using N,N-dimethylformamide as an internal standard. The obtained product was glycerol enriched up to about 90%.

### Nuclear magnetic resonance

Twenty milligrams of the sample were placed in 1.5 mL of D₂O or DMSO-d6 in an NMR tube having an outer diameter of 5 mm. The one-dimensional spectra were recorded in a Varian 400 AS spectrometer at 400 MHz. The acquisition parameters were: spectral width of 6402 Hz, relaxation time of 15 s, number of spectra 128, acquisition time of 4.09 s, pulse width of 90°. The experiments were conducted at 25°C. The spectra were acquired by means of Fourier transform and adjusted with a Gaussian function. A baseline correction was applied in the entire spectral range.

### Enthalpies of fusion and solidification

The equipment used for thermophysical characterization was a DSC-822e calorimeter (Mettler-Toledo). The mass of the sample used was about 15 mg per sample. The sample was introduced in a 100 µL aluminum crucible under an 80 mL min⁻¹ N₂ flow. Measurements were taken in dynamic mode taking into account the melting temperature (Tm) of the sample. A constant increase of 0.5 K min⁻¹ was applied, starting the study 10 K below the Tm of the material until reaching 10 K above the Tm. Mettler-Toledo v.11.00 STARe software was used to evaluate the resulting DSC curves. The enthalpy of phase change and the temperature were obtained by means of integrating the DSC heat flow signal response.

### Example 1. General method for the synthesis of 2-chloro-1-(chloromethyl)ethyl esters (IV)

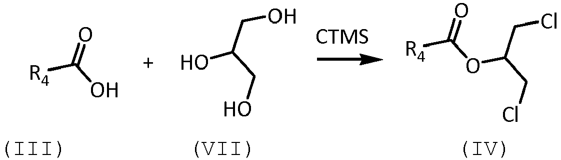

Carboxylic acid (III) (1 mmol), glycerol (VII) (184 mg, 2 mmol) and chlorotrimethylsilane (CTMS, 540 mg, 5 mmol) were added to a reaction vial provided with a PTFE stopper. The mixture was heated at 80°C for 48 h. After cooling, an organic solvent (1 mL of a solvent containing hydrocarbon, such as hexane or pentane, or an ether such as diethyl ether, tetrahydrofuran, t-butyl-methyl-ether or methyltetrahydrofuran) was added and the mixture was washed three times with water. The organic phase was dried over anhydrous MgSO₄ and the solvent was eliminated in vacuo. The residue was purified by crystallization (for solid compounds, using mixtures of ethanol:hydrocarbon solvent or ethyl acetate:hydrocarbon solvent), distillation (liquid compounds with a boiling point lower than 90°C are purified by means of distillation at atmospheric pressure, and liquid compounds with a boiling point between 90°C and 250°C are purified by means of vacuum distillation) or flash column chromatography on silica gel (for those compounds not purified by crystallization or distillation).

Compounds of formula (IV) in which R₄ is (CH₃)₃C-, CH₃-(CH₂)₁₄- and 1-naphthyl were synthesized using this method.

### Example 2. General method for the synthesis of 1,1'-(2-hydroxypropane-1,3-diyl)-bis(3-butyl-1-imidazolium) dichlorides (IX)

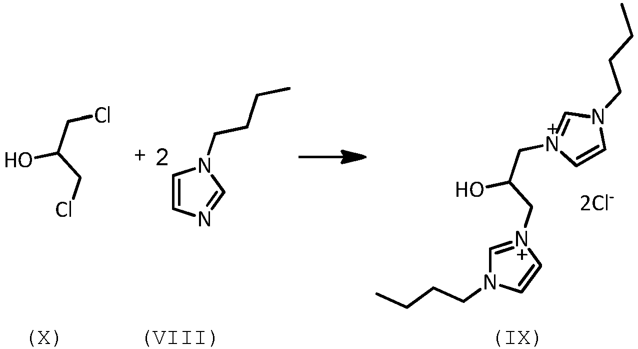

A mixture of 1,3-dichloro-2-propanol ester (X) (50 mmol) and N-butylimidazole (VIII) (12.42 g, 100 mmol) was heated at 110°C for 48 h in a covered reactor under argon atmosphere. The mixture was cooled to room temperature and acetonitrile was added until no more product precipitated. The white solid formed was recovered by means of filtration and washed three times with cold acetonitrile. After drying, a highly hygroscopic product (IX) was recovered. ¹H NMR (400 MHz, DMSO) δ 9.40 (s, 2H), 7.85 (t, *J* = 1.5 Hz, 2H), 7.83 (t, *J* = 1.6 Hz, 2H), 6.38 (d, *J* = 5.9 Hz, 1H), 4.46 (dd, *J* = 13.4, 2.4 Hz, 2H), 4.29-4.19 (m, *J* = 9.5, 4.4 Hz, 1H), 4.20-4.12 (m, *J* = 15.4, 8.0 Hz, 6H), 1.81-1.68 (m, 4H), 1.29-1.17 (m, 4H), 0.86 (t, *J* = 7.4 Hz, 6H).

### Example 3. General method for the synthesis of 1,1'-(2-(alkylcarboxyloxy)propane-1,3-diyl)-bis(3-butyl-1-imidazolium) dichloride (XI)

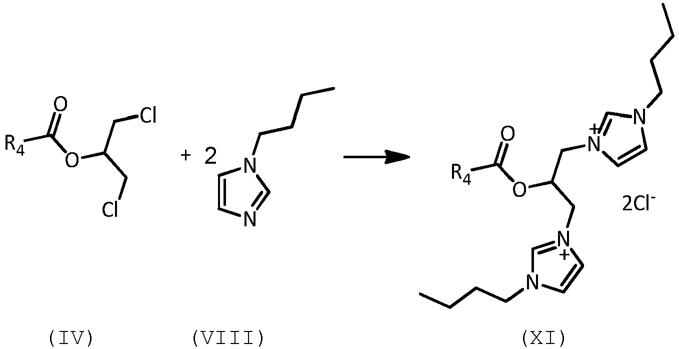

A mixture of a 2-chloro-1-(chloromethyl)ethyl ester (IV) (6.45 g, 50 mmol) and N-butylimidazole (VIII) (12.42 g, 100 mmol) was heated at 110°C for 48 h in a covered reactor under argon atmosphere. The mixture was cooled to room temperature and acetonitrile was added until no more product precipitated. The white solid formed was recovered by means of filtration and washed three times with cold acetonitrile. After drying, 17.36 g of the highly hygroscopic compound (XI) was recovered.

Compounds of formula (XI) in which R₄ is (CH₃)₃C-, CH₃-(CH₂)₁₄- and 1-naphthyl were synthesized using this method.

### Example 4. Synthesis of 1,1'-(2-(acroyloxy)propane-1,3-diyl)-bis(3-butyl-1-imidazolium)dichloride (XIII)

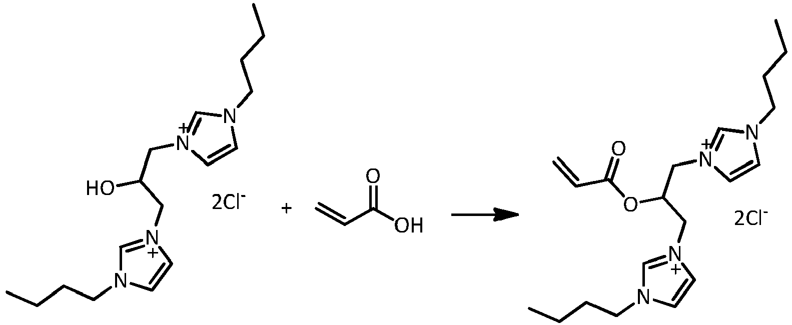

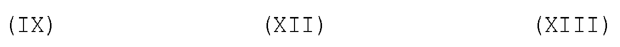

A solution of 80 mL of dichloromethane containing 4 g (55.5 mmol) of acrylic acid (XII), 4.1 g (10.8 mmol) of 1,1'-(2-hydroxypropane-1,3-diyl)bis(3-butyl-1-imidazolium) chloride (IX) and 35.18 mg (0,288 mmol) of 4-dimethylaminopyridine was added to a 250 mL one-necked round-bottom flask equipped with a calcium chloride tube. The solution was cooled in an ice bath, and 6.68 g (32.4 mmol) of DCC were added at 0°C for 5 min under stirring. After another 5 min at 0°C, the ice bath was removed and the reaction mixture was stirred for 24 h at room temperature in a water bath. The reaction mixture was filtered and the precipitate was washed with dry dichloromethane (3x20 mL). The organic solutions were combined and the solvent was evaporated in vacuo. The compound (XIII) was obtained. ¹H NMR (400 MHz, DMSO) δ 9.55 (t, *J* = 1.5 Hz, 2H), 7.92 (t, *J* = 1.8 Hz, 2H), 7.84 (t, *J* = 1.7 Hz, 2H), 6.34-6.14 (m, 3H), 5.69-5.65 (m, 1H), 4.79 (dd, *J* = 14.4, 2.8 Hz, 2H), 4.51 (dd, *J* = 14.5, 8.3 Hz, 2H), 4.18 (t, *J* = 7.0 Hz, 4H), 1.75-1.66 (m, 4H), 1.22-1.09 (m, 4H), 0.83 (t, *J* = 7.4 Hz, 6H).

### Example 5. General method for the synthesis of di(hexafluorophosphate)bisimidazolium derivatives (XIV-XVI)

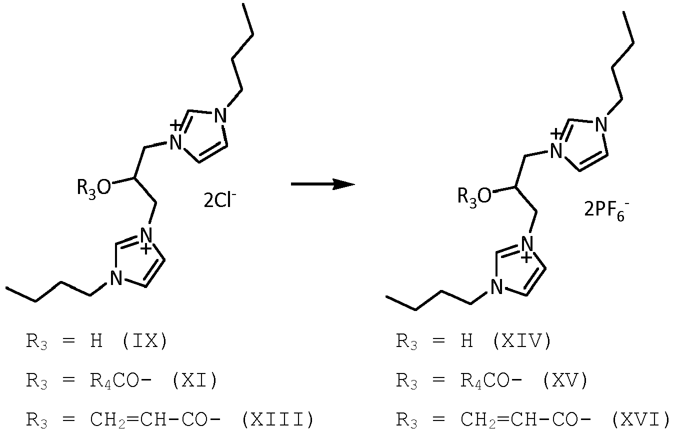

Two mmol of bisimidazolium dichloride (IX, XI, XIII) were dissolved in water (20 mL) and KPF₆ (828.3 mg, 4,5 mmol) was added. The mixture was stirred for 15 min at room temperature. 20 mL of dichloromethane were then added, and the mixture was gently stirred. The organic phase was recovered and dried over anhydrous MgSO₄. The solvent was evaporated in vacuo to yield the corresponding bisimidazolium di(hexafluorophosphate) (XIV-XVI).
Compound (XIV), R₃= H: ¹H NMR (400 MHz, DMSO) δ 9.40 (s, 2H), 7.85 (t, *J* = 1.5 Hz, 2H), 7.83 (t, *J* = 1.6 Hz, 2H), 6.38 (d, *J* = 5.9 Hz, 1H), 4.46 (dd, *J* = 13.4, 2.4 Hz, 2H), 4.29-4.19 (m, *J* = 9.5, 4.4 Hz, 1H), 4.20-4.12 (m, *J* = 15.4, 8.0 Hz, 6H), 1.81-1.68 (m, 4H), 1.29-1.17 (m, 4H), 0.86 (t, *J* = 7.4 Hz, 6H).
Compound (XV) (R₄=(CH₃)₃C-): ¹H NMR (400 MHz, CDCl₃) δ 10.53 (t, *J* = 1.4 Hz, 2H), 8.26 (t, *J* = 1.7 Hz, 2H), 7.22 (t, *J* = 1.8 Hz, 2H), 6.04 (tt, *J* = 8.0, 3.2 Hz, 1H), 5.10 (ddd, *J* = 17.3, 14.1, 5.7 Hz, 4H), 4.31-4.15 (m, 4H), 1.92-1.81 (m, 4H), 1.42-1.30 (m, 4H), 1.09 (s, *J* = 2.3 Hz, 9H), 0.94 (t, *J* = 7.4 Hz, 6H).
Compound (XV) (R₄= CH₃-(CH₂)₁₄-): ¹H NMR (400 MHz, CDCl₃) δ 10.53 (t, *J* = 1.4 Hz, 2H), 8.26 (t, *J* = 1.7 Hz, 2H), 7.22 (t, *J* = 1.8 Hz, 2H), 6.04 (tt, *J* = 8.0, 3.2 Hz, 1H), 5.10 (ddd, *J* = 17.3, 14.1, 5.7 Hz, 4H), 4.31-4.15 (m, 4H), 2.36 (t, *J* = 7.5 Hz, 2H), 1.92-1.81 (m, 4H), 1.64 (q, *J* = 7.4 Hz, 2H), 1.42-1.30 (m, 4H), 1.37-1.20 (m, 24H), 0.94 (t, *J* = 7.4 Hz, 6H), 0.91-0.84 (m, 3H).
Compound (XV) (R₄= 1-naphthyl): ¹H NMR (400 MHz, DMSO) δ 9.56 (s, 2H), 8.48-8.42 (m, *J* = 6.3, 3.4 Hz, 1H), 8.31 (d, *J* = 7.3 Hz, 1H), 8.25 (d, *J* = 8.2 Hz, 1H), 8.05-8.01 (m, *J* = 5.9, 3.7 Hz, 1H), 7.99 (t, *J* = 1.6 Hz, 2H), 7.82 (t, *J* = 1.5 Hz, 2H), 7.64-7.55 (m, 3H), 6.04 (tt, *J* = 8.7, 2.8 Hz, 1H), 4.92 (dd, *J* = 14.4, 2.7 Hz, 2H), 4.69 (dd, *J* = 14.4, 8.6 Hz, 2H), 4.12 (t, *J* = 7.0 Hz, 4H), 1.61-1.51 (m, 4H), 1.03-0.92 (m, 4H), 0.59 (t, *J* = 7.4 Hz, 6H).
Compound (XVI) (R₃= CH₂=CH-CO): ¹H NMR (400 MHz, DMSO-d6) δ 9.24 (s, 2H), 7.83 (m, 2H), 7.78 (m, 2H), 6.41 (dd, J ¼ 17.2, 1.3 Hz, 1H), 6.17 (dd, J ¼ 17.2, 10.5 Hz, 1H), 6.02 (dd, J ¼ 10.5, 1.3 Hz, 1H), 5.64-5.59 (m, 1H), 4.72-4.68 (m, 2H), 4.50-4.44 (m, 2H), 4.24 (q, J ¼ 7.3 Hz, 4H), 1.90 (t, J 7.6 Hz, 4H), 1.40 (m, 4H), 0.92 (t, J 7.4 Hz, 6H).

### Example 6. General method for the synthesis of 1-(2-(acyloxy)-3-chloropropan-1-yl)-3-butylimidazolium chloride and hexafluorophosphate (XI)

A mixture of 2-chloro-1-(chloromethyl)ethyl ester (IV) (50 mmol) and N-butylimidazole (VIII) (50 mmol) was heated at 110°C for 20 h in a reactor under argon atmosphere. The mixture was cooled to room temperature, toluene was added and a white solid was precipitated with chloroform. The desired compound was recovered by means of silica gel column chromatography.

Replacing the chloride counterion for hexafluorophosphate is done by means of the same method described in Example 5, with the only difference being the use of a monoimidazolium chloride:potassium hexafluorophosphate molar ratio of 1:1.2.

### Example 7. Enthalpies of fusion and solidification of bisimidazolium salts and comparison with monoimidazolium salts

| | | | | | | |
|---|---|---|---|---|---|---|
| Bisimidazolium salts | | | | | | |
| 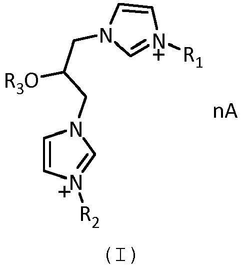 | | | | | | |

| R₁ | R₂ | R₃ | n | A | Enthalpy of fusion (kJ/kg) | Enthalpy of solidification (kJ/kg) |
|---|---|---|---|---|---|---|
| butyl | butyl | (CH₃)₃CCO | 2 | Cl⁻ | 500 | 310 |
| butyl | butyl | (CH₃)₃CCO | 2 | PF₆⁻ | 200 | 150 |
| butyl | butyl | CH₃(CH₂)₁₄CO | 2 | Cl⁻ | 900 | 280 |
| butyl | butyl | CH₃(CH₂)₁₄CO | 2 | PF₆⁻ | 110 | 100 |
| butyl | butyl | H | 2 | Cl⁻ | 400 | 160 |
| butyl | butyl | 1-naphthyl-CO | 2 | Cl⁻ | 350 | 200 |
| butyl | butyl | 1-naphthyl-CO | 2 | PF₆⁻ | 180 | 180 |
| butyl | butyl | CH₂=CH-CO | 2 | Cl⁻ | 650 | 360 |
| butyl | butyl | CH₂=CH-CO | 2 | PF₆⁻ | 250 | 200 |
| Monoimidazolium salts | | | | | | |
| 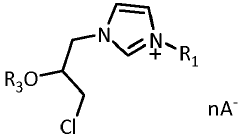 | | | | | | |

| R₁ | | R₃ | n | A⁻ | Enthalpy of fusion (kJ/kg) | Enthalpy of solidification (kJ/kg) |
|---|---|---|---|---|---|---|
| butyl | | (CH₃)₃CCO | 1 | Cl⁻ | 80 | 50 |
| butyl | | (CH₃)₃CCO | 1 | PF₆⁻ | 70 | 50 |

The results show that the bisimidazolium salts of formula (I) that were studied have enthalpies of fusion between 110 kJ/kg and 900 kJ/kg and enthalpies of solidification between 100 kJ/kg and 360 kJ/kg, which are values indicating a significant thermal energy storage capacity which in some cases is much higher than current commercial products. Furthermore, the enthalpies of fusion and solidification of bisimidazolium salts are greater than those of monoimidazolium salts, and as a result bisimidazolium salts have a greater energy storage capacity.

## Claims

1. Use of a compound of formula (I) in thermal energy storage 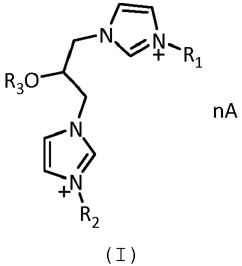 wherein
R₁ and R₂ are independently selected from the group consisting of linear or branched C₁-C₁₂ alkyl, wherein the C₁ alkyl is optionally substituted with one, two or three C₆-C₁₀ aryl groups; linear or branched C₂-C₁₂ alkenyl and C₆-C₁₀ aryl; R₃ is selected from the group consisting of H and R₄CO-;
R₄ is selected from the group consisting of linear or branched C₁-C₂₀ alkyl, wherein the C₁ alkyl is optionally substituted with one, two or three C₆-C₁₀ aryl groups; linear or branched C₂-C₂₀ alkenyl and C₆-C₁₀ aryl;
A is a monovalent or divalent anion; and
n is 2 when A is a monovalent anion or n is 1 when A is a divalent anion.

2. Use according to claim 1, wherein R₁ and R₂ are independently a linear or branched C₁-C₆ alkyl.

3. Use according to any one of claims 1 or 2, wherein R₁ and R₂ are the same.

4. Use according to any one of claims 1 to 3, wherein R₁ and R₂ are butyl.

5. Use according to any one of claims 1 to 4, wherein R₃ is H.

6. Use according to any one of claims 1 to 4, wherein R₃ is R₄CO and R₄ is selected from the group consisting of linear C₁₀-C₂₀ alkyl, branched C₃-C₆ alkyl, linear C₂-C₆ alkenyl, naphthyl and phenyl.

7. Use according to claim 6, wherein R₄ is selected from the group consisting of *tert*-butyl, pentadecanyl, vinyl and naphthyl.

8. Use according to any one of claims 1 to 7, wherein A is a monovalent anion selected from the group consisting of PF₆⁻, Cl⁻, Br⁻ and BF₄⁻.

9. Use according to claim 1, wherein the compound of formula (I) is selected from the group consisting of:
- 1,1'-(2-(2,2-dimethylpropanoyloxy)-1,3-propanediyl)bis(3-butyl-1H-imidazolium)dichloride,
- 1,1'-(2-(2,2-dimethylpropanoyloxy)-1,3-propanediyl)bis(3-butyl-1H-imidazolium)di(hexafluoride),
- 1,1'-(2-palmitoyloxy-1,3-propanediyl)bis(3-butyl-1H-imidazolium) dichloride,
- 1,1'-(2-palmitoyloxy-1,3-propanediyl)bis(3-butyl-1H-imidazolium)di(hexafluoride),
- 1,1'-(2-hydroxy-1,3-propanediyl)bis(3-butyl-1H-imidazolium)dichloride,
- 1,1'-(2-hydroxy-1,3-propanediyl)bis(3-butyl-1H-imidazolium)di(hexafluoride),
- 1,1'-(2-(1-naphthoyloxy)-1,3-propanediyl)bis(3-butyl-1H-imidazolium)dichloride,
- 1,1'-(2-(1-naphthoyloxy)-1,3-propanediyl)bis(3-butyl-1H-imidazolium)di(hexafluoride),
- 1,1'-(2-acroyloxy-1,3-propanediyl)bis(3-butyl-1H-imidazolium)dichloride, and
- 1,1'-(2-acroyloxy-1,3-propanediyl)bis(3-butyl-1H-imidazolium)di(hexafluoride).

10. A thermal energy storage device comprising a compound of formula (I) as defined in any one of claims 1 to 9.

11. The thermal energy storage device according to claim 10, wherein the device is selected from the group consisting of solar panels, boards for walls and ceilings.

12. A compound of formula (II) 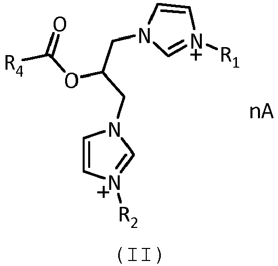 wherein
R₁ and R₂ are independently selected from the group consisting of linear or branched C₁-C₁₂ alkyl, wherein the C₁ alkyl is optionally substituted with one, two or three C₆-C₁₀ aryl groups; linear or branched C₂-C₁₂ alkenyl and C₆-C₁₀ aryl; R₄ is selected from the group consisting of linear or branched C₁-C₂₀ alkyl, wherein the C₁ alkyl is optionally substituted with one, two or three C₆-C₁₀ aryl groups; linear or branched C₃-C₂₀ alkenyl, and C₆-C₁₀ aryl;
A is a monovalent or divalent anion; and
n is 2 when A is a monovalent anion or n is 1 when A is a divalent anion,
where the compound in which R₁ and R₂ are ethyl, R₄ is -C(CH₃)=CH₂ and A is (CF₃SO₂)₂N⁻ is excluded from formula (II) .

13. The compound of formula (II) according to claim 12, wherein R₁ and R₂ are independently a linear or branched C₁-C₆ alkyl.

14. The compound of formula (II) according to any one of claims 12 or 13, wherein R₁ and R₂ are the same.

15. The compound of formula (II) according to any one of claims 12 to 14, wherein R₁ and R₂ are butyl.

16. The compound of formula (II) according to any one of claims 12 to 15, wherein R₄ is selected from the group consisting of linear or branched C₁-C₂₀ alkyl, wherein the C₁ alkyl is optionally substituted with one, two or three C₆-C₁₀ aryl groups; linear C₃-C₂₀ alkenyl, and C₆-C₁₀ aryl.

17. The compound of formula (II) according to any one of claims 12 to 16, wherein R₄ is selected from the group consisting of linear or branched C₁-C₂₀ alkyl, wherein the C₁ alkyl is optionally substituted with one, two or three C₆-C₁₀ aryl groups; and C₆-C₁₀ aryl.

18. The compound of formula (II) according to any one of claims 12 to 17, wherein R₄ is selected from the group consisting of linear C₁₀-C₂₀ alkyl, branched C₃-C₆ alkyl, naphthyl and phenyl.

19. The compound of formula (II) according to any one of claims 12 to 18, wherein R₄ is selected from the group consisting of *tert*-butyl, pentadecanyl and naphthyl.

20. The compound of formula (II) according to any one of claims 12 to 19, wherein A is a monovalent anion selected from the group consisting of PF₆⁻, Cl⁻, Br⁻ and BF₄⁻.

21. The compound of formula (II) according to any one of claims 12 to 20, wherein the compound is selected from the group consisting of:
- 1,1'-(2-(2,2-dimethylpropanoyloxy)-1,3-propanediyl)bis(3-butyl-1H-imidazolium)dichloride,
- 1,1'-(2-(2,2-dimethylpropanoyloxy)-1,3-propanediyl)bis(3-butyl-1H-imidazolium)di(hexafluoride),
- 1,1'-(2-palmitoyloxy-1,3-propanediyl)bis(3-butyl-1H-imidazolium) dichloride,
- 1,1'-(2-palmitoyloxy-1,3-propanediyl)bis(3-butyl-1H-imidazolium)di(hexafluoride),
- 1,1'-(2-(1-naphthoyloxy)-1,3-propanediyl)bis(3-butyl-1H-imidazolium)dichloride, and
- 1,1'-(2-(1-naphthoyloxy)-1,3-(propanediyl)bis(3-butyl-1H-imidazolium)di(hexafluoride).

22. A method for obtaining a compound of formula (II) as defined in any of claims 12 to 21, which comprises:
a) reacting glycerol with a carboxylic acid of formula (III) in the presence of a chlorinating agent
R₄-COOH (III)
to yield a compound of formula (IV) 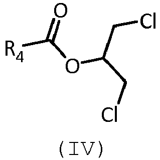 wherein R₄ is as defined in any one of claims 12 and 16 to 19,
b) reacting the compound of formula (IV) with a compound of formula (V) and a compound of formula (VI), 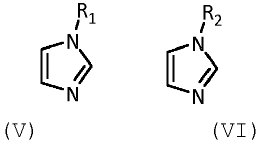
wherein R₁ and R₂ are as defined in any of claims 12 to 15, and when R₁=R₂, then compounds (V) and (VI) are the same compound;
to yield a compound of formula (II), 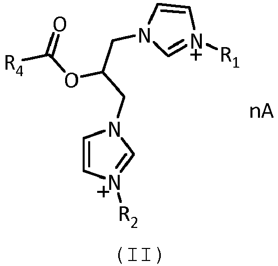 wherein R₁, R₂ and R₄ are as defined in any of claims 12 to 19; n is 2, and A is Cl⁻;
c) optionally treating the compound of formula (II) with a salt of A to yield a compound of formula (II) wherein A is a monovalent or divalent anion as defined in any one of claims 12 or 20 other than Cl⁻.

23. The method according to claim 22, wherein the chlorinating agent of step a) is selected from the group consisting of chlorotrimethylsilane, CaCl₂ in the presence of H₃PO₄, and AlCl₃ optionally in the presence of H₃PO₄.
